# EUROPEAN PATENT SPECIFICATION

(11) **EP 1 545 467 B1**
(45) Date of publication and mention of the grant of the patent: **12.09.2007**
(21) Application number: 03750572.4
(22) Date of filing: 18.09.2003
(51) Int. Cl.: A61K 9/16, A61K 9/20, A61K 31/4184

(54) **SOLID PHARMACEUTICAL FORMULATIONS COMPRISING TELMISARTAN**
FESTE PHARMAZEUTISCHE FORMULIERUNGEN MIT TELMISARTAN
FORMULATIONS PHARMACEUTIQUES SOUS FORME SOLIDE CONTENANT DU TELMISARTAN

(30) Priority: 24.09.2002 DE 10244681
(43) Date of publication of application: 29.06.2005
(73) Proprietor: Boehringer Ingelheim International GmbH, 55216 Ingelheim am Rhein (DE)
(72) Inventor: NAKATANI, Manabu, Hyogo 666-0037 (JP); OHKI, Toshimitsu, Osaka 563-0043 (JP); TAKESHI, Sawada, Osaka 563-0043 (JP); TOYOSHIMA, Kenzo, Ikoma-shi, Nara-ken 630-0254 (JP)
(86) International application number: PCT/EP2003/010382
(87) International publication number: WO 2004/028505

(56) References cited:
- WO-A-00/27397
- WO-A-03/059327
- FR-A- 2 787 330

## Description

### FIELD OF THE INVENTION

The present invention relates to new solid pharmaceutical compositions comprising the angiotensin II receptor antagonist telmisartan, e.g. in form of granules or in form of a powder, as well as solid oral formulations ready for use/ingestion, e.g. capsule and tablet formulations made from said pharmaceutical compositions. The present invention also provides methods for producing said compositions and formulations.

### BACKGROUND OF THE INVENTION

INN Telmisartan is an angiotensin II receptor antagonist developed for the treatment of hypertension and other medical indications as disclosed in EP-A-502314. its chemical name is 4'-[2-n-propyl-4-methyl-6-(1-methylbenzimidazol-2-yl)-benzimidazol-1-ylmethyl]-biphenyl-2-carboxylic acid having the following structure:

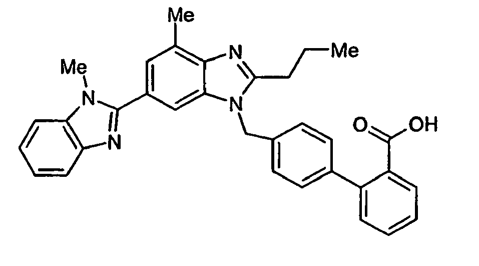

Telmisartan is generally manufactured and supplied in the free acid form. As disclosed in WO 00/43370, crystalline telmisartan exists in two polymorphic forms having different melting points. Under the influence of heat and humidity, the lower melting polymorph B transforms irreversibly into the higher melting polymorph A. Both forms are characterized by a very poor solubility in aqueous systems at the physiological pH range of the gastro-intestinal tract of between pH 1 to 7.

Telmisartan is obtainable on the market under the trade name Micardis^{®}. Starting from the free acid form Telmisartan as introduced to the market is manufactured using an expensive spray-drying process. WO 00/27397 discloses additional spray-dried granules comprising telmisartan, and tablets comprising these granules. Due to the poor solubility of the free acid form preparation of alternative telmisartan formulation is difficult.

### BRIEF SUMMARY OF THE INVENTION

There is a clear need to provide alternative solid oral formulations of Telmisartan which can be prepared using less complicated and expensive processes and fulfill all prerequisites for pharmaceutical use, i.e. long-lasting stability of the formulation under different climatic conditions and sufficient solubility of the active substance for sufficient gastrointestinal absorption in the slightly acidic and neutral pH region.

It is a first object of the invention to provide said alternative solid pharmaceutical compositions comprising telmisartan, e.g. in form of granules or a powder, in a form allowing that the active compound is released with sufficient solubility for gastrointestinal absorption in the slightly acidic and neutral pH region from said compositions and formulations.

Preferably, the formulations should have immediate release characteristics and a dissolution showing no essential pH dependency within the physiological relevant pH interval of the gastrointestinal tract.

It is a second object of the invention to provide further solid oral formulations ready for use/ingestion, e.g. capsule and tablet formulations, made from said pharmaceutical compositions mentioned under the first aspect of the invention.

A third object of the invention relates to methods for producing said compositions and formulations mentioned hereinbefore.

### DETAILED DESCRIPTION OF THE INVENTION

### First object of the invention (pharmaceutical composition)

Surprisingly it has been found that the solubility of telmisartan can be raised by a factor of several hundreds by a pharmaceutical composition comprising 3 to 50 wt.% of telmisartan dispersed in a dissolving matrix comprising
(a) a basic agent in a molar ratio of basic agent: telmisartan = 1:1 to 10:1,
(b) a surfactant or emulsifier in an amount of about 1 to 20 wt.% of the final composition,
(c) 25 to 70 wt.% of a water-soluble diluent, and
(d) optionally 0 to 20 wt.% of further excipients and/or adjuvants,
the sum of all components adding to 100%.

The term "dissolving tablet matrix" refers to a pharmaceutical tablet base formulation having immediate release (fast dissolution) characteristics that readily dissolves in a physiological aqueous medium.

Specific examples of suitable basic agents are alkali metal hydroxides such as NaOH and KOH; furthermore NaHCO₃, KHCO₃, Na₂CO₃, K₂CO₃, Na₂HPO₄, K₂HPO₄; basic amino acids such as arginine; and meglumine (N-methyl-D-glucamine).

The surfactants and emulsifiers may be ionic or non-ionic, the latter being preferred. Specific examples of surfactants and emulsifiers are such as poloxamers or pluronics, polyethylene glycols, polyethylene glycol monostearate, polysorbates, sodium lauryl sulfate, polyethoxylated and hydrogenated castor oil etc.

With regard to the poloxamers or pluronics suitable as non-ionic surfactants and emulsifiers is referred to the definition given in The Merck Index, 12^{th} edition, 1996. Suitable poloxamers may have an average mol weight of about 2000 to 12000, preferably 4000 to 10000, more preferred 6000 to 10000, most preferred 8000 to 9000. Examples for specific poloxamers are poloxamer 182LF, poloxamer 331 and poloxamer 188.

Specific examples of suitable water-soluble diluents are carbohydrates such as monosaccharides like glucose; oligosaccharides like sucrose; and sugar alcohols like erythritol, sorbitol, mannitol, dulcitol, ribitol and xylitol. Mannitol, erythritol, sorbitol and sucrose are preferred diluents.

The other excipients and/or adjuvants are, for instance, selected from binders, carriers, lubricants, flow control agents, crystallization retarders, solubilizers and coloring agents.

The binder may be selected from the group of dry binders and/or the group of wet granulation binders, depending on the manufacturing process chosen for the pharmaceutical composition. Suitable dry binders are, e.g., cellulose powder, crystalline cellulose, microcrystalline cellulose or light anhydrous silicic acid. Specific examples of wet granulation binders are corn starch, polyvinyl pyrrolidone (Povidone), vinylpyrrolidone-vinylacetate copolymer (Copovidone) and cellulose derivatives like hydroxymethylcellulose, hydroxyethylcellulose, hydroxypropyl-cellulose and hydroxypropylmethylcellulose.

Suitable disintegrants are, e.g., sodium starch glycolate, Crospovidon, Croscarmellose, sodium carboxymethylcellulose and dried corn starch.

The other excipients and adjuvants, if used, are preferably selected from diluents and carriers such as cellulose powder, crystalline cellulose or microcrystalline cellulose, cellulose derivatives like hydroxymethylcellulose, hydroxyethylcellulose, hydroxypropylcellulose and hydroxy-propylmethylcellulose, dibasic calcium phosphate, corn starch, pregelatinized starch, polyvinyl pyrrolidone (Povidone) etc.; lubricants such as stearic acid, magnesium stearate, sodium stearylfumarate, glycerol tribehenate, etc.; flow control agents such as colloidal silica, light anhydrous silicic acid, crystalline cellulose, talc, etc.; crystallization retarders such as Povidone, etc.; coloring agents, including dyes and pigments such as Iron Oxide Red or Yellow, titanium dioxide, talc, etc.; and mixtures of two or more of these excipients and/or adjuvants.

The pharmaceutical compositions according to the present invention provide improved solubilization of the poorly water-soluble telmisartan of up to a concentration of more than 4.4 mg/100mL, thereby facilitating dissolution of the drug at a physiological pH level, and also provides for immediate release from the fast disintegrating matrix.

The presence of component (b), a surfactant or emulsifier, is essential to achieve a substantially improved dissolution of the active ingredient as well as for the use of a simplified manufacture process such as fluid-bed granulation instead of spray-drying for preparing the solid pharmaceutical compositions according to the invention.

In a preferred embodiment the pharmaceutical composition according to the invention comprises 10 to 35 wt.% of telmisartan dispersed in a dissolving matrix comprising
(a) a basic agent, in a molar ratio of basic agent : telmisartan = 1.5:1 to 5:1,
(b) a non-ionic surfactant or emulsifier, in an amount of about 1 to 10 wt.% of the final composition,
(c) 35 to 60 wt.% of a water-soluble diluent, and
(d) optionally 0 to 20 wt.% of further excipients and/or adjuvants,
the sum of all components adding to 100%.

All specified components (a) to (d) mentioned hereinbefore may be used in the preferred embodiment, whereas
preferred basic agents are NaOH, KOH, arginine and meglumine,
preferred non-ionic surfactants or emulsifiers are selected from poloxamers, polyethylene glycols, polyethoxylated and hydrogenated castor oil,
preferred water-soluble diluents are selected from sucrose, erythritol, sorbitol, mannitol and xylitol, and
preferred optional further excipients and/or adjuvants are selected from crystalline cellulose, light anhydrous silicic acid, hydroxymethylcellulose, hydroxyethylcellulose, hydroxypropyl-cellulose, hydroxypropylmethylcellulose, magnesium stearate, corn starch, polyvinyl pyrrolidone, vinylpyrrolidone-vinylacetate copolymer, stearic acid, magnesium stearate, sodium stearylfumarate, colloidal silica, talc, povidone and coloring agents.

In a more preferred embodiment the pharmaceutical composition according to the invention comprises 15 to 25 wt.% of telmisartan dispersed in a dissolving matrix comprising
(a) a basic agent, in a molar ratio of basic agent: telmisartan = 2:1 to 3:1,
(b) a non-ionic surfactant or emulsifier, in an amount of about 2 to 7 wt.% of the final composition ,
(c) 35 to 50 wt.% of a water-soluble diluent, and
(d) optionally 0 to 20 wt.% of further excipients and/or adjuvants,
the sum of all components adding to 100%.

All specified components (a) to (d) mentioned hereinbefore may be used in the more preferred embodiment, whereas
the most preferred basic agent is meglumine,
the most preferred non-ionic surfactants are selected from poloxamers,
the most preferred water-soluble diluents are selected from mannitol, erythritol, sorbitol and sucrose, and
the most preferred optional further excipients and/or adjuvants are selected from crystalline cellulose, light anhydrous silicic acid and magnesium stearate.

In any embodiment of the invention one or more of the non-ionic surfactants or emulsifiers, water-soluble diluents and excipients and/or adjuvants may be present.

### Second object of the invention (formulation ready for use/ingestion)

A second object of the invention is directed to solid oral formulations ready for use/ingestion, e.g. capsule and tablet formulations made from the pharmaceutical compositions mentioned hereinbefore. Capsule formulations can be obtained by simply filling the powdery or granulated pharmaceutical formulations mentioned hereinbefore in conventional capsules, for instance hard or soft gelatine capsules. Tablet formulations also can be prepared by conventional techniques, for instance by direct compression of the powdery or granular pharmaceutical compositions mentioned hereinbefore.

The tablets so obtained can be further processed using conventional techniques, for instance can be coated using suitable coatings known in the art which do not negatively affect the dissolution properties of the final formulation. For instance the tablets can be provided with a seal coat for moisture protection by melting a high molecular weight polyethylene glycol or any polyethylene glycol which is solid at room temperature (25°C) onto the core tablets. Even though the polymer is water soluble, its rate of solution is slow enough to afford the core tablets moisture protection. Other polymers, which offer similar water solubility and a similar degree of moisture protection may also be used.

Additionally, agents such as beeswax, shellac, cellulose acetate phthalate, polyvinyl acetate phthalate, zein, film forming polymers such as hydroxypropyl cellulose, ethylcellulose and polymeric methacrylates can be dissolved in a suitable solvent and applied to the tablets, provided that the coating has no substantial effect on the disintegration/dissolution of the dosage form and that the coated dosage form is physiochemically stable.
After the dosage form is sealed, a sugar coating may be applied onto the sealed pharmaceutical dosage form. The sugar coating may comprise sucrose, dextrose, sorbitol and the like or mixtures thereof. If desired, colorants or opacifiers may be added to the sugar solution.

Composition of both, capsule and (core) tablet formulations, is preferably the same as mentioned hereinbefore with respect to the pharmaceutical formulations. In the alternative, additional amounts of the optional excipients and/or adjuvants mentioned hereinbefore can be added before filling the powdery or granulated pharmaceutical formulations into capsules or compressing them to tablets, e.g. in order to adjust the concentration of the active compound to a certain value (for instance by adding more filler), to improve flowability of powdery formulations, to improve compressibility (for instance by adding more lubricant or binder), or other routine process optimization known to the skilled person.

The solid oral formulations according to the present invention generally contain 10 to 160. mg, preferably 20 to 80 mg, of telmisartan. Presently preferred forms comprise 20,40 or 80 mg of telmisartan, respectively.

For instance, the total composition of capsule and tablet formulations according to the invention may vary within the following ranges, with the proviso that the proportional composition given above with respect to the basic pharmaceutical compositions is met:
10 to 160 mg of telmisartan;
10 to 160 mg of meglumine or arginine, or
2 to 33 mg of NaOH, or
3 to 46 mg of KOH, or
4 to 80 mg of NaHCO₃, KHCO₃, Na₂CO₃, K₂CO₃, Na₂HPO₄ or K₂HPO₄;
2 to 40 mg of non-ionic surfactants or emulsifiers;
20 to 200 mg of water soluble diluents; and
0 to 80 mg, of further excipients and/or adjuvants;
preferably
20 to 80 mg of telmisartan;
20 to 80 mg of meglumine, or
4 to 16 mg of NaOH, or
6 to 23 mg of KOH;
4 to 20 mg of non-ionic surfactants or emulsifiers selected from poloxamers, polyethylene glycols, polyethoxylated and hydrogenated castor oil, poloxamers being especially preferred;
40 to 100 mg of water soluble diluents selected from glucose, sucrose, erythritol, sorbitol, mannitol and xylitol; and
0.2 to 40 mg of further excipients and/or adjuvants selected from crystalline cellulose, light anhydrous silicic acid, hydroxymethylcellulose, hydroxyethylcellulose, hydroxypropyl-cellulose , hydroxypropylmethylcellulose, magnesium stearate, corn starch, polyvinyl pyrrolidone, vinylpyrrolidone-vinylacetate copolymer, stearic acid, magnesium stearate, sodium stearylfumarate, colloidal silica, talc, povidone and coloring agents;
most preferred
35 to 45 mg of telmisartan;
35 to 45 mg of meglumine;
6 to 10 mg of non-ionic surfactants or emulsifiers selected from poloxamers, poloxamer 188 being especially preferred;
70 to 90 mg of water soluble diluents selected from mannitol, erythritol, sorbitol and sucrose; and
0.2 to 20 mg of further excipients and/or adjuvants selected from crystalline cellulose, light anhydrous silicic acid and magnesium stearate.

It should be understood that for capsule formulations it may be of advantage to add a flow control agent such as colloidal silica, light anhydrous silicic acid, crystalline cellulose before filling the capsule on a capsule filling machine with the powdered pharmaceutical composition in order to improve flow properties of the composition. Therefore, in the capsule formulations the content of the further excipients and/or adjuvants will preferably be in the upper range, e.g. in the range of 10 to 20 wt.% of the total formulation. In contrast, flow control agents are preferably not added for production of tablet formulations according to the present invention since these agents, in combination with the high compression forces used in tablet production, deteriorate dissolution or disintegration of the tablets. Therefore, in tablet formulations the content of the further excipients and/or adjuvants will preferably be in the lower range, e.g. in the range of 0.1 to 5 wt.%, preferably 0.3 to 2 wt.%, of the final formulation since only low amounts of lubricants should be present

The tablet formulations according to the present invention can also be used for preparation of fixed dose combination products, for instance together with a diuretic as the second active component. Suitable diuretics are thiazide and thiazide-analogue diuretics like hydrochlorothiazide (HCTZ), clopamide, xipamide or chlorotalidone, and any other diuretic suitable in the treatment of hypertension like, e.g., furosemide and piretanide, and combinations thereof with amiloride and triamteren. HCTZ is incompatible with basic agents being a component of the telmisartan tablet formulations according to the invention. This problem can be overcome by means of a bilayer pharmaceutical tablet comprising a first telmisartan containing tablet layer prepared from a pharmaceutical composition mentioned hereinbefore under the first aspect of the invention, and a second tablet layer containing a diuretic in a disintregrating tablet matrix.

The second tablet layer composition generally comprises 1.5 to 35 wt.%, preferably 2 to 15 wt.%, of active ingredient; 25 to 75 wt.%, preferably 35 to 65 wt.%, of filler; 10 to 40 wt.%, preferably 15 to 35 wt.%, of dry binder; 0.5 to 5 wt.%, preferably 1 to 4 wt.%, of wet granulation binder, and 1 to 10 wt.%, preferably 2 to 8 wt.%, of disintegrant. The other excipients and adjuvants are generally employed in the same amount as in the first tablet layer composition. The filler is may be selected from anhydrous lactose, spray-dried lactose and lactose monohydrate.

Tablets of the present invention tend to be very low hygroscopic and may be packaged using PVC-blisters, PVDC-blisters or a moisture-proof packaging material such as aluminium foil blister packs, polypropylene tubes, glass bottles and HDPE bottles.

### Third object of the invention (process for preparing pharmaceutical compositions and final formulations)

The third object of the invention is directed to methods for producing the solid pharmaceutical compositions mentioned hereinbefore. The compositions comprising telmisartan according to the invention may be prepared by any suitable method known to those skilled in the art, for instance, by freeze drying of aqueous solutions, coating of carrier particles in a fluidized bed, and by solvent deposition on sugar pellets or other carriers. Preferably, however, the pharmaceutical compositions are prepared using a granulation process, e.g. the fluid-bed granulation process (A), or, in the alternative, the spray-drying process (B) described specifically hereinafter. The less complicated and cheaper fluid-bed granulation process (A) is preferred.

Since during subsequent processing telmisartan is normally dissolved and transformed into a substantially amorphous form, its initial crystal morphology and particle size are of little importance for the physical and biopharmaceutical properties of the pharmaceutical composition obtained.

In a first embodiment a fluid-bed granulation process (A) can be used for preparation of the pharmaceutical compositions according to the invention, characterized by the following steps:
(i) preparing a granulation liquid as an aqueous solution by dissolving 3 to 50 wt.% of telmisartan together with the following components in water or in a mixture solution of ethanol and water:
   (a) a basic agent in a molar ratio of basic agent telmisartan = 1:1 to 10:1,
   (b) a non-ionic surfactant or emulsifier in an amount of about 1 to 20 wt.%,
(ii) placing 25 to 70 wt.% of a water-soluble diluent into a fluid-bed granulator, optionally together with 10 to 20 wt.% of a dry binder, including a premix-step,
(iii) carrying out the fluid-bed granulation using the granulation liquid for spraying onto the components placed into the granulator,
(iv) after completion of the granulation drying and, optionally, screening the granulate obtained,
(v) optionally blending the granulate with further excipients and/or adjuvants in order to prepare the final composition, and
(vi) optionally milling the granulate thus obtained in order to produce a powdery composition of defined particle size distribution;
wherein all percentage amounts given are related to the final composition to be prepared.

Preferred embodiments of the process with regard to specific components and proportional amounts fully correspond to those disclosed hereinbefore with regard to the first aspect of the invention.

In the premix step of step (ii) an inlet air temperature of about 60 to 120°C may be used. In the granulation step (iii) step an inlet temperature of about 80 to 100°C may be used. The spraying rate greatly depends on the type of granulator used as well as the batch size and can be adjusted by the skilled person by routine. Only for instance, a spraying rate of 400 to 1000 mL/min may be suitable for a 200 kg granulate batch. Lower or higher spray rates may also used.
In the drying step of step (iv) an inlet temperature of about 60 to 120°C, and a duration of drying of about 1 to 30 minutes may be used. In the screening step a screen with a mesh size of 0.5 to 3 mm may be suitable.
The optional milling step (vi) can be carried out conventionally by the skilled person.

In a second embodiment a spray-drying process (B) can be used for preparation of the pharmaceutical compositions according to the invention, characterized by the following steps:
(i) preparing an aqueous spray-solution by dissolving 3 to 50 wt.% of telmisartan together with the following components in water or mixture solution of ethanol and water:
   (a) a basic agent in a molar ratio of basic agent: telmisartan = 1:1 to 10:1,
   (b) a non-ionic surfactant or emulsifier in an amount of about 1 to 20 wt.%,
(ii) spray-drying said aqueous spray-solution to obtain a spray-dried granulate;
(iii) mixing said spray-dried granulate with 25 to 70 wt.% of a water-soluble diluent to optain a premix;
(iv) optionally, mixing said premix with a lubricant;
(v) optionally, adding further excipients and/or adjuvants in any of steps (i) to (iv),
wherein all percentage amounts given are related to the final composition to be prepared.

If it is necessary to adjust a particular particle size distribution in a powdery composition thus obtained a conventional milling step may be applied, preferably before optional addition of a lubricant according to step (iv). Furthermore, a powdery composition may be converted into a granular composition applying conventional granulation techniques.

Preferred embodiments of the process with regard to specific components and proportional amounts fully correspond to those disclosed hereinbefore with regard to the first aspect of the invention.

In a preferred embodiment of process (B), an aqueous alkaline solution of telmisartan is prepared by dissolving the active ingredient in water or mixture solution of ethanol and water with the help of one or more basic agents like sodium hydroxide or meglumine. Optionally, a recrystallization retarder may be added. The dry matter content of the starting aqueous solution is generally 10 to 40 wt.%, preferably 20 to 30 wt.%.

The aqueous solution is then spray-dried at room temperature or preferably at increased temperatures of, for instance, between 50 and 100°C in a co-current or countercurrent spray-drier at a spray pressure of, for instance, 1 to 4 bar. Generally speaking, the spray-drying conditions are preferably chosen in such a manner that a spray-dried granulate having a residual humidity of ≤ 5 wt.%, preferably ≤ 3.5 wt.%, is obtained in the separation cyclone. To that end, the outlet air temperature of the spray-drier is preferably kept at a value of between about 80, and 90°C while the other process parameters such as spray pressure, spraying rate, inlet air temperature, etc. are adjusted accordingly.
The spray-dried granulate obtained is preferably a fine powder having the following particle size distribution:
- d₁₀:: ≤ 20 µm, preferably ≤ 10 µm
- d₅₀:: ≤ 80 µm, preferably 20 to 55 µm
- d₉₀:: ≤ 350 µm, preferably 50 to 150 µm

After spray-drying, the active ingredient (telmisartan) as well as the excipients contained in the spray-dried granulate are in a substantially amorphous state with no crystallinity being detectable. From a physical point of view, the spray-dried granulate is a solidified solution or glass having a glass transition temperature Tg of preferably > 50°C, more preferably > 80°C.
The lubricant is generally added to the premix in an amount of 0.1 to 5 wt.%, preferably 0.3 to 2 wt.%, based on the weight of the final composition.
Mixing is carried out in two stages, i.e. in a first mixing step the spray-dried granulate and the diluent are admixed using , e.g., a high-shear mixer or a free-fall blender, and in a second mixing step the lubricant is blended with the premix, preferably also under conditions of high shear. The method of the invention is however not limited to these mixing procedures and, generally, alternative mixing procedures may be employed in any steps of the process comprising a mixing procedure, such as, e.g., container mixing with intermediate screening.

Batches of granulates with different composition obtained by process (A) or (B) may be blended together in order to adjust a target composition and may additionally be blended with further excipients and/or adjuvants such as lubricants, if required for adjusting a final composition for further processing into the final formulation ready for use/ingestion, for instance for filling into capsules using a suitable capsule filling machine or for direct compression of tablets using a suitable rotary tablet press.

For direct compression, the final composition may be prepared by dry-mixing the constituent components, e.g. by means of a high-intensity mixer or a free-fall blender. Alternatively, the final composition may be prepared using a wet granulation technique wherein an aqueous solution of a wet granulation binder is added to a premix and subsequently the wet granulate obtained is dried, e.g. in a fluidized-bed dryer or drying chamber. The dried mixture is screened and then a lubricant is admixed, e.g. using a tumbling mixer or free-fall blender, whereafter the composition is ready for compression.

A bilayer-tablet mentioned under the second aspect of the invention can be prepared by the following process:
(i) providing a first tablet layer composition comprising telmisartan by use of the fluid-bed granulation process (A) or the spray-drying process (B) described hereinbefore,
(ii) providing a second tablet layer composition by
   a) mixing and/or granulating a diuretic with the constituents of a disintegrating tablet matrix and, optionally, further excipients and/or adjuvants;
   b) admixing a lubricant to obtain a final blend for the second tablet layer;
(iii) introducing the first or the second tablet layer composition in a tablet press;
(iv) compressing said tablet layer composition to form a tablet layer;
(v) introducing the other tablet layer composition into the tablet press; and
(vi) compressing both tablet layer compositions to form a bilayer tablet.

For preparing the bilayer tablet according to the present invention, the first and second tablet layer compositions may be compressed in the usual manner in a bilayer tablet press, e.g. a high-speed rotary press in a bilayer tableting mode.

However, care should be taken not to employ an excessive compression force for the first tablet layer. Preferably, the ratio of the compression force applied during compression of the first tablet layer to the compression force applied during compression of both the first and second tablet layers is in the range of from 1:10 to 1:2. For instance, the first tablet layer may be compressed at moderate force of 4 to 8 kN, whereas the main compression of first plus second layer is performed at a force of 10 to 20 kN.

During bilayer tablet compression adequate bond formation between the two layers is achieved by virtue of distance attraction forces (intermolecular forces) and mechanical interlocking between the particles.

In order to avoid any cross-contamination between the first and second tablet layers (which could lead to decomposition of HTCZ), any granulate residues have to be carefully removed during tableting by intense suction of the die table within the tableting chamber.

### Release study of the active ingredient

The solid oral formulations of the present invention release the active ingredient telmisartan rapidly and with minor pH dependency. Normally, at least 70% and typically at least 80% of the drug load are dissolved after 30 min and release of the major fraction occurring within less than 20 min.

Table 1 shows a typical capsule formulation containing a pharmaceutical composition according to the invention, designated formulation A, containing as the non-ionic surfactant or emulsifier 8 mg of Poloxamer 188 (polyoxyethlene[160]polyoxypropylene[30]glycol) and a corresponding reference formulation, designated formulation B, containing instead of the Poloxamer component additional 8 mg of D-mannitol. The dissolution of these capsule formulations was evaluated in aqueous solutions of pH 1.2 and 4.0 according to JP paddle method, 100prm, 900mL, 37°C, dissolution medium: pH 1.2 JP 1^{st} fluid, pH 4.0 acetic-acid buffer, detection: UV/296 nm.

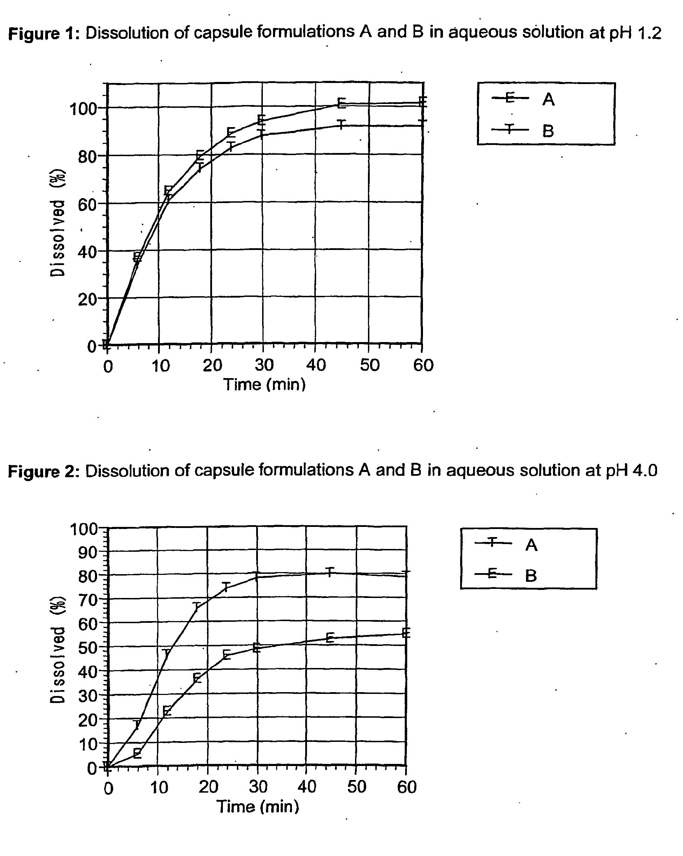

The results obtained can be seen in Figure 1 and 2 showing release of the active ingredient telmisartan given as "dissolution %". The release of telmisartan was essentially faster in the presence of Poloxamer 188.

**Table 1: Composition of tested capsule formulations**

| Formulation | A | B |
|---|---|---|
| Telmisartan | 40 mg | 40 mg |
| Meglumine | 40 mg | 40 mg |
| Poloxamer 188 | 8 mg | 0 mg |
| D-Mannitol | 81 mg | 89 mg |
| Crystalline cellulose (Avicel PH101) | 30 mg | 30 mg |
| Magnesium stearate | 1 mg | 1 mg |
| Total | 200 mg/cap | 200 mg/cap |

### SHORT DESCRIPTION OF THE DRAWINGS

Figure 1 shows the release profile of the active ingredient telmisartan from capsule formulation A according to the invention in comparison to a corresponding capsule formulation B without the Poloxamer 188 component in aqueous test solution (JP.1^{st} fluid) at pH 1.2,
Figure 2 shows the release profile of telmisartan from formulation A in comparison to formulation B in aqueous acetic-acid buffer at pH 4.0.

In order to further illustrate the present invention, the following non-limiting examples are given:

The following table shows solid pharmaceutical compositions according to the invention. Formulations C, D, E, F and G are granular formulations which can be filled in capsules, formulations D, E, F and G also can be compressed to form tablets. All formulations contain 40 mg of telmisartan, whereas alternative capsule and tablet formulations containing 20 or 80 mg of telmisartan are homologues formulations.

**Table 2:**

| Formulation | C | D | E | F | G |
|---|---|---|---|---|---|
| Telmisartan | 40.0 mg | 40.0 mg | 40.0 mg | 40.0 mg | 40.0 mg |
| Meglumine | 40.0 mg | 40.0 mg | 40.0 mg | 40.0 mg | 40.0 mg |
| Poloxamer 188 | 8.0 mg | 8.0 mg | 8.0 mg | 8.0 mg | 8.0 mg |
| D-mannitol | 81.5 mg | 80.6 mg | - | 70.6 mg | - |
| Erythritol | - | - | 80.5 mg | - | - |
| Sorbitol | - | - | - | 10.0 mg | - |
| Sucrose | - | - | - | - | 80.6 mg |
| Crystalline cellulose | 30.0 mg | - | - | - | - |
| Light anhydrous silicic acid | - | - | 0.1 mg | - | - |
| Magnesium stearate | 0.5 mg | 1.4 mg | 1.4 mg | 1.4 mg | 1.4 mg |
| Total | 200.0 mg | 170.0 mg | 170.0 mg | 170.0 mg | 170.0 mg |

### Manufacturing:

### 1. Granulation liquid or spray-solution

90 kg of purified water are measured into a suitable stainless steel vessel at a temperature of between 20-40°C. In sequence, 8 kg of Poloxamer 188 (polyoxyethlene[160]polyoxypropylene[30]glycol), 40 mg of meglumine and 40 kg of telmisartan (mixture of polymorph A and B) are dissolved in the purified water under intensive stirring until a virtually clear solution is obtained. Total volume is about 160 L.

### 2. Granulation

### Alternative (a) for producing Formulation C:

81.5 kg of D-mannitol and 30 kg of crystalline cellulose (e.g. Avicel PH101 or 302) are placed into a fluid-bed granulator, briefly pre-mixed and sprayed with 178 kg of granulation liquid (containing 88 kg of dry mass). Then is sprayed with 2 L of purified water, followed by a drying step and a screening step.

### Alternative (b) for producing Formulation D :

80.6 kg of D-mannitol are placed into a fluid-bed granulator and sprayed with 178 kg of granulation liquid (containing 88 kg of dry mass). The granulation mass is then sprayed with 2 L of purified water, follwed by a drying step and a screening step.

### Alternative (c) for producing Formulation E:

80.5 kg of erythritol and 0.1kg of light anhydrous silicic acid are placed into a fluid-bed granulator and sprayed with 178 kg of granulation liquid (containing 88 kg of dry mass). Then is sprayed with 2 L of purified water; followed by a drying step and a screening step.

### Alternative (d) for producing Formulation F:

70.6 kg of erythritol and 10 kg of sorbitol are placed into a fluid-bed granulator and sprayed with 178 kg of granulation liquid (containing 88 kg of dry mass). Then is sprayed with 2 L of purified water, follwed by a drying step and a screening step.

### Alternative (e) for producing Formulation G:

80.6 kg of sucrose are placed into a fluid-bed granulator and sprayed with 178 kg of granulation liquid (containing 88 kg of dry mass). Then is sprayed with 2 L of purified water, followed by a drying step and a screening step.

### Process data pre-mixing:

Inlet air temperature: 80 - 100 °C
End of pre-mixing: Gut temperature about 55 °C

### Process data granulation:

Inlet air temperature: 80-100 °C
Spraying rate: 500-900 mL/min

### Process data drying step:

Inlet air temperature: 80 -100 °C
End of drying: Gut temperature more than 70 °C
Duration of drying: about 5 minutes

### Process data screening step:

The granules are screened, for instance using an oscillator or comil screen machine, with a mesh size of 1.5 mm.

### 3. Final mixture for preparation of capsule formulation:

Two 199.5 kg batches of screened granules produced according to granulation alternative (a) are mixed using a suitable mixer with a revolution of 10 rpm for 10 to 20 min, resulting in a 399 kg mixed batch which is finally blended with 1 kg of magesium stearate, using a suitable mixer with a revolution of 10 rpm for about 15 min thus producing the final mixture.

### 4. Final mixture for preparation of tablet formulation:

Two 199.5 kg batches of screened granules produced according to granulation alternative (b), (c), (d) or (e) are mixed using a suitable mixer with a revolution of 10 rpm for 10 to 20 min, resulting in a 399 kg mixed batch which is finally blended with 1 kg of magesium stearate, , using a suitable mixer with a revolution of 10 rpm for about 15 min thus producing the final mixture.

### 5. Capsule filling

The final mixture for capsule formulation is filled into capsules using a suitable capsule filling machine (100, 200 or 400 mg per capsule).

### 6. Tablet compression

Using a suitable rotary tablet press the final mixture for tablet compression is compressed into tablets. The target weight is 85, 170 or 340 mg.

Process parameters for tabletting:

| Tablet press | Fette 3090 |
|---|---|
| Tabletting speed | 100.000 (80.000 - 120.000) tabl./h |
| Stirrer blade speed: | about 30 rpm |
| Compression force | 7 (5 - 10) KN |

The tablet hardness can be adjusted by variation of the main compression force.

### 8. Production of a spray-dried formulation

The spray-solution described above is sprayed into a suitable spray dryer, e.g. a Niro P 6.3 equipped with Schlick atomizing nozzles of 1.0 mm diameter, with a flow-through heating coil connected upstream of the dryer, and dried to give a white to off-white fine granulate. The spray mode is counter-current at a spray-pressure of about 3 bar, an inlet air temperature of about 125°C and a spray rate of about 11 kg/h, thus resulting in an outlet air temperature of about 85°C. The temperature of the flow through heating coil water bath is set at a temperature of about 80°C.

88 kg of the spray-dried granules are mixed with 80.6 kg of powdered D-mannitol using a suitable mixer with a revolution of 10 rpm for about 15 min and finally blended with 1.4 kg of magnesiumstearate thus producing the final mixture ready for filling into capsules or compression into tablets.

## Claims

1. A pharmaceutical composition comprising 3 to 50 wt.% of telmisartan dispersed in a dissolving matrix comprising
(a) a basic agent in a molar ratio of basic agent : telmisartan = 1:1 to 10:1,
(b) a surfactant or emulsifier in an amount of 1 to 20 wt.% of the final composition,
(c) 25 to 70 wt.% of a water-soluble diluent, and
(d) optionally 0 to 20 wt.% of further excipients and/or adjuvants,
the sum of all components adding to 100%.

2. The pharmaceutical composition of claim 1 wherein the basic agent is a metal hydroxide such as NaOH and KOH; or is selected from NaHCO_{3,} KHCO_{3,} Na₂CO_{3,} K₂CO₃, Na₂HPO₄, K₂HPO₄, from basic amino acids such as arginine; and from meglumine (N-methyl-D-glucamine).

3. The pharmaceutical composition of claim 1 wherein the surfactants and emulsifiers are selected from poloxamers or pluronics, polyethylene glycols, polyethylene glycol monostearate, polysorbates, sodium lauryl sulfate, polyethoxylated and hydrogenated castor oil.

4. The pharmaceutical composition of claim 1 wherein wherein the surfactants and emulsifiers are selected from poloxamers having an average mol weight of 2000 to 12000.

5. The pharmaceutical composition of claim 4 wherein the poloxamer is selected from poloxamer 182LF, poloxamer 331 and poloxamer 188.

6. The pharmaceutical composition of claim 1 wherein the water-soluble diluents are selected from carbohydrates such as monosaccharides like glucose; oligosaccharides like sucrose; and sugar alcohols like erythritol, sorbitol, mannitol, dulcitol, ribitol and xylitol.

7. The pharmaceutical composition of claim 1 wherein the other excipients and/or adjuvants are selected from binders, carriers, lubricants, flow control agents, crystallization retarders, solubilizers and coloring agents.

8. A solid oral pharmaceutical formulation ready for use/ingestion produced from a pharmaceutical composition of any of claims 1 to 7.

9. The formulation of claim 8 in form of a capsule or a tablet.

10. A formulation of claim 8 or 9 comprising a dosage unit of 10 to 160 mg of telmisartan.

11. A bilayer pharmaceutical tablet comprising a first telmisartan containing tablet layer prepared from a pharmaceutical composition of any of claims 1 to 7 and a second tablet layer containing a diuretic in a disintregrating tablet matrix.

12. Process for preparing the pharmaceutical composition of claim 1 using a fluid-bed granulation process (A), **characterized by** the following steps:
(i) preparing a granulation liquid as an aqueous solution by dissolving 3 to 50 wt.% of telmisartan together with the following components in water or in a mixture solution of ethanol and water:
(a) a basic agent in a molar ratio of basic agent : telmisartan = 1:1 to 10:1,
(b) a non-ionic surfactant or emulsifier in an amount of 1 to 20 wt.%,
(ii) placing 25 to 70 wt.% of a water-soluble diluent into a fluid-bed granulator, optionally together with 10 to 20 wt.% of a dry binder, including a premix-step,
(iii) carrying out the fluid-bed granulation using the granulation liquid for spraying onto the components placed into the granulator,
(iv) after completion of the granulation drying and, optionally, screening the granulate obtained,
(v) optionally blending the granulate with further excipients and/or adjuvants in order to prepare the final composition, and
(vi) optionally milling the granulate thus obtained in order to produce a powdery composition of defined particle size distribution;
wherein all percentage amounts given are related to the final composition to be prepared.

13. Process for preparing the pharmaceutical composition of claim 1 using a spray-drying process (B), **characterized by** the following steps:
(i) preparing an aqueous spray-solution by dissolving 3 to 50 wt.% of telmisartan together with the following components in water or mixture solution of ethanol and water:
(a) a basic agent in a molar ratio of basic agent : telmisartan = 1:1 to 10:1,
(b) a non-ionic surfactant or emulsifier in an amount of 1 to 20 wt.%,
(ii) spray-drying said aqueous spray-solution to obtain a spray-dried granulate;
(iii) mixing said spray-dried granulate with 25 to 70 wt.% of a water-soluble diluent to optain a premix;
(iv) optionally, mixing said premix with a lubricant;
(v) optionally, adding further excipients and/or adjuvants in any of steps (i) to (iv),
wherein all percentage amounts given are related to the final composition to be prepared.

14. Process for preparing the bilayer tablet of claim 11 using the following steps:
(i) providing a first tablet layer composition comprising telmisartan by use of the process of claim 12 or 13,
(ii) providing a second tablet layer composition by
a) mixing and/or granulating a diuretic with the constituents of a disintegrating tablet matrix and, optionally, further excipients and/or adjuvants;
b) admixing a lubricant to obtain a final blend for the second tablet layer;
(iii) introducing the first or the second tablet layer composition in a tablet press;
(iv) compressing said tablet layer composition to form a tablet layer;
(v) introducing the other tablet layer composition into the tablet press; and
(vi) compressing both tablet layer compositions to form a bilayer tablet.

## Patentansprüche

1. Pharmazeutische Zusammensetzung, umfassend 3 bis 50 Gew.-% Telmisartan, dispergiert in einer Lösungsmatrix, umfassend:
(a) ein basisches Mittel in einem molaren Verhältnis basisches Mittel:Telmisartan= 1: 1 bis 10:1,
(b) ein oberflächenaktives Mittel oder ein Emulgator in einer Menge von 1 bis 20 Gew.-% der Endzusammensetzung,
(c) 25 bis 70 Gew.-% eines wasserlöslichen Verdünnungsmittels und
(d) gegebenenfalls 0 bis 20 Gew.-% weiterer Hilfsstoffe und/oder Zusatzstoffe,
wobei sich die Summe sämtlicher Komponenten auf 100% addiert.

2. Pharmazeutische Zusammensetzung nach Anspruch 1, worin das basische Mittel ein Metallhydroxid darstellt, wie NaOH und KOH; oder ausgewählt ist aus NaHCO₃, KHCO₃, Na₂CO₃, K₂CO₃, Na₂HPO₄, K₂HPO₄, aus basischen Aminosäuren, wie Arginin; und aus Meglumin (N-Methyl-D-glucamin).

3. Pharmazeutische Zusammensetzung nach Anspruch 1, worin die oberflächenaktiven Mittel und Emulgatoren ausgewählt sind aus Poloxameren oder Pluronics, Polyethylenglykolen, Polyethylenglykolmonostearat, Polysorbaten, Natriumlaurylsulfat, polyethoxyliertem und hydriertem Castoröl.

4. Pharmazeutische Zusammensetzung nach Anspruch 1, worin die oberflächenaktiven Mittel und Emulgatoren aus Poloxameren mit einem mittleren Molgewicht von 2000 bis 12000 ausgewählt sind.

5. Pharmazeutische Zusammensetzung nach Anspruch 4, worin das Poloxamer aus Poloxamer 182LF, Poloxamer 331 und Poloxamer 188 ausgewählt ist.

6. Pharmazeutische Zusammensetzung nach Anspruch 1, worin die wasserlöslichen Verdünnungsmittel ausgewählt sind aus Kohlenhydraten, wie zum Beispiel Monosacchariden, wie Glucose; Oligosacchariden, wie Saccharose; und Zuckeralkoholen, wie Erythritol, Sorbitol, Mannitol, Dulcitol, Ribitol und Xylitol.

7. Pharmazeutische Zusammensetzung nach Anspruch 1, worin die anderen Hilfsstoffe und/oder Zusatzstoffe ausgewählt sind aus Bindemitteln, Trägern, Schmiermitteln, Fließkontrollmitteln, Kristallisationsverzögerern, Löslichkeitsvermittlern und Farbstoffen.

8. Feste orale pharmazeutische Formulierung, fertig zur Verwendung/Aufnahme, hergestellt aus einer pharmazeutischen Zusammensetzung nach irgendeinem der Ansprüche 1 bis 7.

9. Formulierung nach Anspruch 8 in Form einer Kapsel oder einer Tablette.

10. Formulierung nach Anspruch 8 oder 9, umfassend eine Dosierungseinheit von 10 bis 160 mg Telmisartan.

11. Zweilagige pharmazeutische Tablette, umfassend eine erste Telmisartan enthaltende Tablettenschicht, hergestellt aus einer pharmazeutischen Zusammensetzung nach irgendeinem der Ansprüche 1 bis 7, und eine zweite Tablettenschicht, enthaltend ein Diuretikum in einer sich auflösenden Tablettenmatrix.

12. Verfahren zur Herstellung der pharmazeutischen Zusammensetzung nach Anspruch 1, unter Verwendung eines Fließbettgranulationsverfahrens (A), **gekennzeichnet durch** die folgenden Schritte:
(i) Herstellen einer Granulationsflüssigkeit als eine wässerige Lösung **durch** Auflösen von 3 bis 50 Gew.-% Telmisartan zusammen mit den folgenden Komponenten in Wasser oder einer gemischten Lösung aus Ethanol und Wasser:
(a) einem basischen Mittel in einem molaren Verhältnis basisches Mittel:Telmisartan=1:1 bis 10:1,
(b) einem nichtionischen oberflächenaktiven Mittel oder Emulgator in einer Menge von 1 bis 20 Gew.-%,
(ii) Platzieren von 25 bis 70 Gew.-% eines wasserlöslichen Verdünnungsmittels in einen Fließbettgranulator, gegebenenfalls zusammen mit 10 bis 20 Gew.-% eines Trockenbindemittels, einschließlich eines Vormischungsschritts,
(iii) Durchführen der Fließbettgranulation unter Verwendung der Granulationsflüssigkeit zum Sprühen auf die im Fließbettgranulator platzierten Komponenten,
(iv) nach Abschluß der Granulation Trocknen und gegebenenfalls Sichten bzw. Sieben des erhaltenen Granulats,
(v) gegebenenfalls Mischen des Granulats mit weiteren Hilfsstoffen und/oder Zusatzstoffen, um die Endzusammensetzung herzustellen, und
(vi) gegebenenfalls Mahlen des so erhaltenen Granulats, um eine pulverige Zusammensetzung mit einer definierten Partikelgrößenverteilung herzustellen;
worin sich sämtliche angegebenen Prozentwerte auf die herzustellende Endzusammensetzung beziehen.

13. Verfahren zur Herstellung der pharmazeutischen Zusammensetzung nach Anspruch 1 unter Verwendung eines Sprühtrocknungsverfahrens (B), **gekennzeichnet durch** die nachfolgenden Schritte:
(i) Herstellen einer wässerigen Sprühlösung **durch** Auflösen von 3 bis 50 Gew.-% Telmisartan zusammen mit den folgenden Komponenten in Wasser oder einer gemischten Lösung aus Ethanol und Wasser:
(a) einem basischen Mittel in einem molaren Verhältnis basisches Mittel:Telmisartan=1:1 bis 10:1,
(b) einem nichtionischen oberflächenaktiven Mittel oder Emulgator in einer Menge von 1 bis 20 Gew.-%,
(ii) Sprühtrocknen der wässerigen Sprühlösung, um ein sprühgetrocknetes Granulat zu erhalten;
(iii) Mischen des sprühgetrockneten Granulats mit 25 bis 70 Gew.-% eines wasserlöslichen Verdünnungsmittels, um eine Vormischung zu erhalten;
(iv) gegebenenfalls Mischen der Vormischung mit einem Schmiermittel; (v) gegebenenfalls Zugeben weiterer Hilfsstoffe und/oder Zusatzstoffe in irgendeinem der Schritte (i) bis (iv),
worin sich sämtliche angegebenen Prozentwerte auf die herzustellende Endzusammensetzung beziehen.

14. Verfahren zur Herstellung der zweilagigen Tablette nach Anspruch 11 unter Verwendung der nachfolgenden Schritte:
(i) Bereitstellen einer ersten Tablettenschichtzusammensetzung, umfassend Telmisartan, unter Verwendung des Verfahrens nach Anspruch 12 oder 13,
(ii) Bereitstellen einer zweiten Tablettenschichtzusammensetzung durch
a) Mischen und/oder Granulieren eines Diuretikums mit den Bestandteilen einer sich auflösenden Tablettenmatrix und gegebenenfalls weiterer Hilfsstoffe und/oder Zusatzstoffe;
b) Hinzumischen eines Schmiermittels, um eine Endmischung für die zweite Tablettenschicht zu erhalten;
(iii) Einführen der ersten oder zweiten Tablettenschichtzusammensetzung in eine Tablettenpresse;
(iv) Komprimieren der Tablettenschichtzusammensetzung, um eine Tablettenschicht zu bilden;
(v) Einführen der anderen Tablettenschichtzusammensetzung in die Tablettenpresse und
(vi) Komprimieren beider Tablettenschichtzusammensetzungen, um eine zweilagige Tablette zu bilden.

## Revendications

1. Composition pharmaceutique comprenant 3 à 50 % en masse de telmisartan dispersé dans une matrice à dissolution comprenant
(a) un agent basique dans un rapport molaire agent basique : telmisartan = 1 : 1 à 10 : 1,
(b) un tensioactif ou émulsifiant en une quantité de 1 à 20 % en masse de la composition finale,
(c) 25 à 70 % en masse d'un diluant soluble dans l'eau, et
(d) éventuellement 0 à 20 % en masse d'autres excipients et/ou adjuvants,
la somme de tous les composants constituant 100 %.

2. Composition pharmaceutique selon la revendication 1 où l'agent basique est un hydroxyde métallique comme NaOH et KOH ; ou est choisi parmi NaHCO₃, KHCO₃, Na₂CO₃, K₂CO₃, Na₂HPO₄, K₂HPO₄, parmi des aminoacides basiques comme l'arginine ; et parmi la méglumine (N-méthyl-D-glucamine).

3. Composition pharmaceutique selon la revendication 1 où les tensioactifs et émulsifiants sont choisis parmi les poloxamères ou pluronics, les polyéthylèneglycols, le monostéarate de polyéthylèneglycol, les polysorbates, le laurylsulfate de sodium, l'huile de ricin polyéthoxylée et hydrogénée.

4. Composition pharmaceutique selon la revendication 1 où les tensioactifs et émulsifiants sont choisis parmi les poloxamères ayant une masse moléculaire moyenne de 2000 à 12000.

5. Composition pharmaceutique selon la revendication 4 où le poloxamère est choisi parmi le poloxamère 182LF, le poloxamère 331 et le poloxamère 188.

6. Composition pharmaceutique selon la revendication 1 où les diluants solubles dans l'eau sont choisis parmi les glucides tels que les monosaccharides comme le glucose ; les oligosaccharides comme le saccharose ; et les alcools de sucres comme l'érythritol, le sorbitol, le mannitol, le dulcitol, le ribitol et le xylitol.

7. Composition pharmaceutique selon la revendication 1 où les autres excipients et/ou adjuvants sont choisis parmi les liants, les supports, les lubrifiants, les agents de régulation de l'écoulement, les retardateurs de cristallisation, les solubilisants et les agents colorants.

8. Formulation pharmaceutique orale solide prête à l'emploi/ingestion produite à partir d'une composition pharmaceutique selon l'une quelconque des revendications 1 à 7.

9. Formulation selon la revendication 8 sous forme d'une capsule ou d'un comprimé.

10. Formulation selon la revendication 8 ou 9 comprenant une unité de prise de 10 à 160 mg de telmisartan.

11. Comprimé pharmaceutique bicouche comprenant une première couche de comprimé contenant du telmisartan préparée à partir d'une composition pharmaceutique selon l'une quelconque des revendications 1 à 7 et une seconde couche de comprimé contenant un diurétique dans une matrice de comprimé à désintégration.

12. Procédé pour préparer la composition pharmaceutique selon la revendication 1 au moyen d'un procédé de granulation en lit fluidisé (A) **caractérisé par** les étapes suivantes :
(i) préparation d'un liquide de granulation sous forme d'une solution aqueuse par dissolution de 3 à 50 % en masse de telmisartan en même temps que les composants suivants dans l'eau ou dans une solution de mélange d'éthanol et d'eau :
(a) un agent basique dans un rapport molaire agent basique : telmisartan = 1 : 1 à 10: 1,
(b) un tensioactif ou émulsifiant non ionique en une quantité de 1 à 20 % en masse,
(ii) mise en place de 25 à 70 % en masse d'un diluant soluble dans l'eau dans un granulateur à lit fluidisé, éventuellement en même temps que 10 à 20 % en masse d'un liant sec, incluant une étape de prémélange,
(iii) conduite de la granulation en lit fluidisé en utilisant le liquide de granulation pour pulvériser sur les composants placés dans le granulateur,
(iv) après la fin de la granulation séchage et, éventuellement, tamisage des granulés obtenus,
(v) éventuellement mélange des granulés avec d'autres excipients et/ou adjuvants pour préparer la composition finale, et
(vi) éventuellement broyage des granulés ainsi obtenus pour produire une composition pulvérulente de distribution de taille des particules définie ;
où toutes les quantités en pourcentage données sont rapportées à la composition finale destinée à être préparée.

13. Procédé pour préparer la composition pharmaceutique selon la revendication 1 au moyen d'un procédé de séchage par pulvérisation (B) **caractérisé par** les étapes suivantes :
(i) préparation d'une solution aqueuse de pulvérisation par dissolution de 3 à 50 % en masse de telmisartan en même temps que les composants suivants dans l'eau ou dans une solution de mélange d'éthanol et d'eau :
(a) un agent basique dans un rapport molaire agent basique : telmisartan = 1 : 1 à 10 : 1,
(b) un tensioactif ou émulsifiant non ionique en une quantité de 1 à 20 % en masse,
(ii) séchage par pulvérisation de ladite solution aqueuse de pulvérisation pour obtenir des granulés séchés par pulvérisation ;
(iii) mélange desdits granulés séchés par pulvérisation avec 25 à 70 % en masse d'un diluant soluble dans l'eau pour obtenir un prémélange ;
(iv) éventuellement, mélange dudit prémélange avec un lubrifiant ;
(v) éventuellement, addition d'autres excipients et/ou adjuvants dans l'une quelconque des étapes (i) à (iv) ;
où toutes les quantités en pourcentage données sont rapportées à la composition finale destinée à être préparée.

14. Procédé pour préparer le comprimé bicouche selon la revendication 11 au moyen des étapes suivantes :
(i) fourniture d'une composition pour première couche de comprimé comprenant du telmisartan en utilisant le procédé selon la revendication 12 ou 13,
(ii) fourniture d'une composition pour seconde couche de comprimé en
a) mélangeant et/ou granulant un diurétique avec les constituants d'une matrice de comprimé à désintégration et, éventuellement, d'autres excipients et/ou adjuvants ;
b) mélangeant un lubrifiant pour obtenir un mélange final pour la seconde couche de comprimé ;
(iii) introduction de la composition pour première ou seconde couche de comprimé dans une presse à comprimés ;
(iv) compression de ladite composition pour couche de comprimé pour former une couche de comprimé ;
(v) introduction de l'autre composition pour couche de comprimé dans la presse à comprimés ; et
(vi) compression des deux compositions pour couche de comprimé pour former un comprimé bicouche.
